# EUROPEAN PATENT APPLICATION

(11) **EP 4 603 089 A1**
(43) Date of publication of application: **20.08.2025**
(21) Application number: 23877324.6
(22) Date of filing: 12.10.2023
(51) Int. Cl.: A61K 31/198, A23L 33/10, A61K 31/54, A61P 1/16, A61P 9/00, A61P 13/12, A61P 25/00, A61P 25/28, A61P 43/00

(54) **SIRTUIN ACTIVATOR OR EXPRESSION ENHANCER, NAD+ INCREASING AGENT, AND SENESCENT CELL INHIBITOR**

(30) Priority: 12.10.2022 JP 2022164311
(71) Applicant: Wakunaga Pharmaceutical Co., Ltd., Osaka-shi, Osaka 532-0003 (JP)
(72) Inventor: SUZUKI Junichiro, Akitakata-shi, Hiroshima 739-1195 (JP); NAKAMOTO Masato, Akitakata-shi, Hiroshima 739-1195 (JP)
(74) Representative: Ström & Gulliksson AB
(86) International application number: PCT/JP2023/036962
(87) International publication number: WO 2024/080323

(57) **Abstract**

Provided is a novel use of a composition that contains a prescribed sulfur-containing compound, a salt thereof, or both.

The present invention is a sirtuin activator or expression enhancer, a NAD+ increasing agent, or a senescent cell inhibitor containing, as an active ingredient, a compound (excluding S-1-propenylcysteine) represented by general formula 1A or 1B, or a salt thereof.

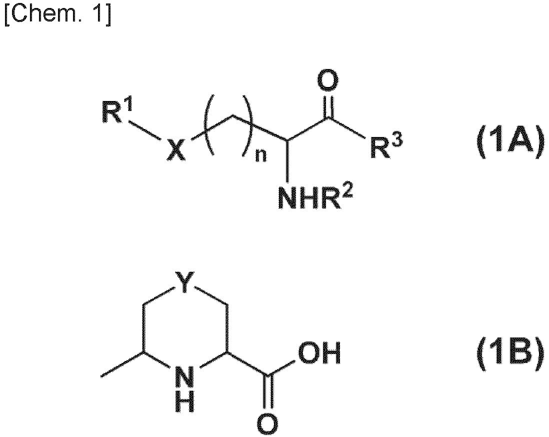

## Description

### TECHNICAL FIELD

The present invention relates to an agent for use in activation or expression enhancement of a sirtuin, NAD⁺ increase, and senescent cell suppression.

### BACKGROUND ART

Sirtuins are known as molecules, for example, in nematodes, flies, and mammals, whose high expression can prolong lifespan while deficiency thereof can shorten such lifespan. Genes of sirtuins that are NAD⁺ dependent deacetylases are widely conserved from bacteria to eukaryotes. Sirtuins have been attracting attention as candidates for longevity genes in animals, because deficiency of Sir2, which is a sirtuin homolog of yeast and nematodes, shortens the lifespan, while overexpression of Sir2 prolongs the lifespan. There are seven sirtuins (SIRT1 to SIRT7) in mammals, and among them, SIRT1 having a structure and function that is the most similar to yeast Sir2 has been revealed to be probably involved in controlling a wide range of cellular functions such as gene expression associated with aging, intracellular metabolism, energy consumption, inflammation, and stress response pathways (Non-Patent Literature 1). In recent years, it has been revealed that sirtuins may be related to specific diseases. For example, Non-Patent Literature 2 shows that cognitive functions including immediate memory, classical conditioning, and spatial learning are impaired in SIRT1 knockout mice. Conversely, overexpression of SIRT1 is observed to exhibit ordered synaptic plasticity and memory. It has been revealed that SIRT1 acts on normal learning, memory, and synaptic plasticity. Furthermore, Non-Patent Documents 3 and 4 describe relationships of SIRT1 with liver steatosis and cardiac function respectively.

Nicotinamide mononucleotide (NAD⁺) functions as a coenzyme for dehydrogenases and a substrate for sirtuins in living bodies. It is also known that an increase in NAD⁺ amount enhances sirtuins activity. Synthesis of NAD is controlled by nicotinamide phosphoribosyltransferase (NAMPT). It is known that decreased function of NAMPT with age leads to reduced NAD⁺ amount and decreased sirtuins activity. Non-Patent Document 5 discloses that administration of nicotinamide riboside, which is a precursor of NAD⁺, suppresses cellular senescence in brain. Non-Patent Documents 6 and 7 describe relationships of NAD⁺ with nonalcoholic fatty liver diseases and kidney injuries respectively.

Cellular senescence is a phenomenon of the occurrence of irreversible cell cycle arrest that results from excessive DNA damage via accumulation of DNA replication errors associated with cell division, oxidative stress, radiation, activation of oncogenes, or the like, which activates p16/RB pathways and p53/p21 pathways, inducing inhibitors of cyclin inhibitor kinase. Cells that have undergone the cellular senescence (the senescent cells) accumulate in tissues due to accelerated cellular senescence and reduced removal ability caused by decline in mitochondria and immune functions associated with aging. The senescent cells accumulated in tissues secrete, for example, inflammatory cytokines, proteases, etc., which are called cellular senescence associated secretory factors, thereby damaging surrounding tissues and accelerating aging of tissues and living bodies. On the other hand, it has been suggested that removal of senescent cells may prevent or delay tissue dysfunction to suppress the senescence (Non-Patent Document 8). Furthermore, it has been revealed that an increase in senescent cells is involved in kidney injuries (Non-Patent Document 9) and fatty liver (Non-Patent Document 10).

On the other hand, it is known that sulfur-containing components present in natural plants of the genus Arium, particularly garlic, etc. can have various biological functions. Examples of such components include S-alkyl(alkenyl)mercaptocysteine (Non-Patent Document 11), S-methylcysteine sulfoxide (Non-Patent Document 12), and S-allylcysteine sulfoxide (Non-Patent Document 13). However, sirtuin activation, senescent cell suppression, and NAD⁺ increase by known sulfur-containing components are not known at all.

### Citation List

### Non-Patent Document

Non-Patent Document 1: Trends Cell Biol. 2014; 24(8): pp. 464-71
Non-Patent Document 2: J Neurosci 2010;30(29): pp. 9695-9707
Non-Patent Document 3: Molecular Medicine Reports 2018; 18: pp. 1609-1615
Non-Patent Document 4: Aging 2021; 13(10): pp. 14482-14498
Non-Patent Document 5: Cell Metab. 2018 Mar 6; 27(3): pp. 513-528
Non-Patent Document 6: British Journal of Pharmacology 2016; 173: pp. 2352-2368
Non-Patent Document 7: Nature 2016; 531 (7595): pp. 528-532
Non-Patent Document 8: Nature. 2011 Nov 2; 479 (7372): pp. 232-6
Non-Patent Document 9: Front Pharmacol. 2019; 10: 770
Non-Patent Document 10: Nature Communications 2017; 8: 15691
Non-Patent Document 11: J. Agric. Food Chem. 2013; 61: pp. 1896-1903
Non-Patent Document 12: Tissue Cell 2021; 69: 101483
Non-Patent Document 13: International Journal of Molecular Medicine 2019; 44: pp. 1943-1951

### DISCLOSURE OF THE INVENTION

### Problems to be Solved by the Invention

The present invention relates to provision of a novel use of a predetermined sulfur-containing compound or a salt thereof, or a composition containing the same.

### Means for Solving the Problems

The present invention relates to the following 1) to 11).
1) An agent for sirtuin activation or sirtuin expression enhancement, an agent for increasing NAD⁺, or an agent for suppressing senescent cells, the agent including a compound represented by the following general formula 1A or 1B (excluding S-1-propenylcysteine) or a salt thereof as an active ingredient: 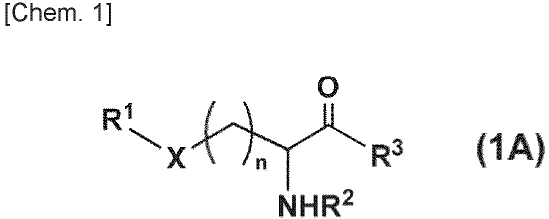
   wherein R¹ represents an alkyl group that may have a substituent, an alkenyl group that may have a substituent, or an alkynyl group that may have a substituent, R² represents a hydrogen atom or an acyl group, R³ represents a hydroxy group or a monosubstituted amino group, X represents a group represented by -S-, -S(O)-, -S(O)₂-, -S-S-, -S-S-S-, or -S-S-S-S-, and n represents 1 or 2, and 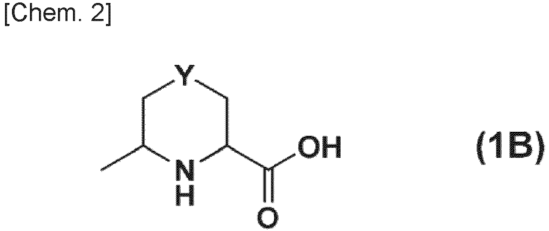
   wherein Y represents a group represented by -S- or -S(O)-.
2) The agent as described in 1), in which in the general formula 1A,
   R¹ represents a C1 to C8 alkyl group that may have a substituent, a C2 to C8 alkenyl group that may have a substituent, or a C2 to C8 alkynyl group that may have a substituent,
   R² represents a hydrogen atom, a C1 to C3 alkylcarbonyl group, or a residue obtained by removing -OH from a carboxy group of an amino acid, and
   R³ represents a hydroxy group or a residue obtained by removing one hydrogen atom from an amino group of an amino acid.
3) The agent as described in 2), in which in the general formula 1A,
   R¹ represents a C1 to C8 alkyl group, a hydroxy C1 to C8 alkyl group, a C2 to C8 alkenyl group, or a C2 to C8 alkynyl group,
   R² represents a hydrogen atom,
   R³ represents a hydroxy group, and
   X represents a group represented by -S-, -S(O)- or -S-S-.
4) The agent as described in 1), in which in the formula 1B,
   Y represents a group represented by -S-.
5) The agent as described in 1), in which the compound is a compound represented by any of the following structural formulae.

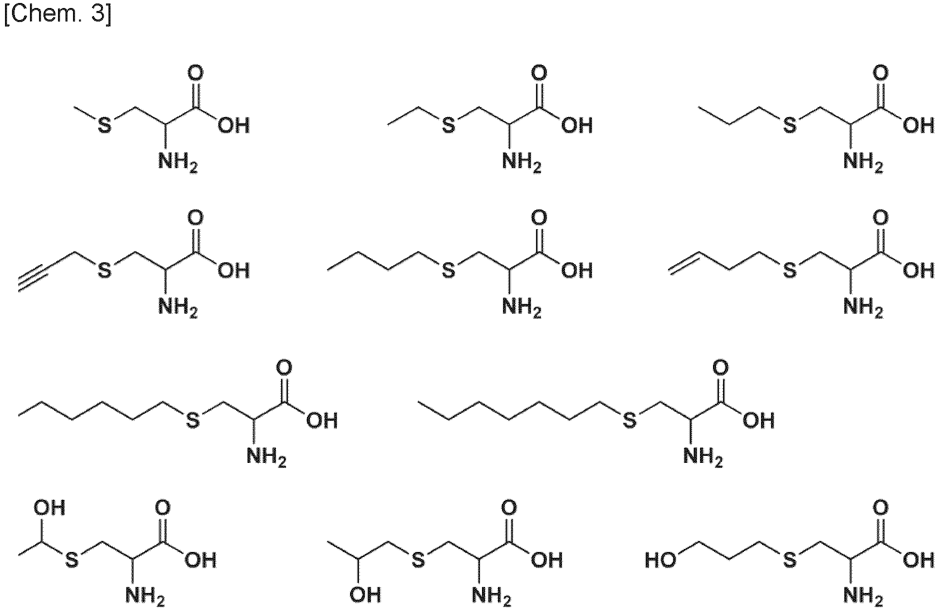

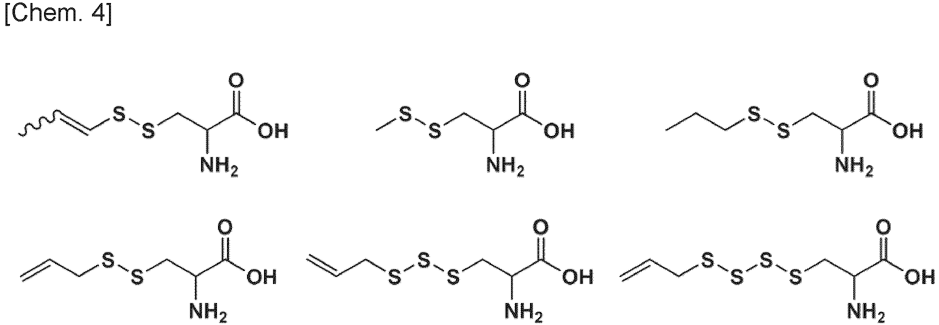

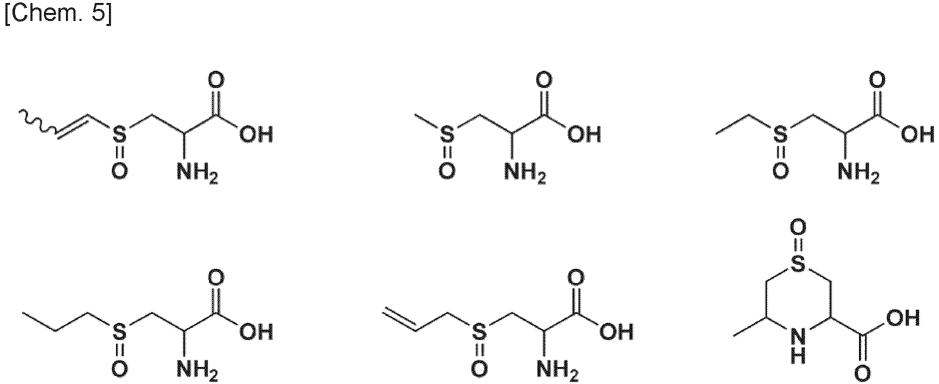

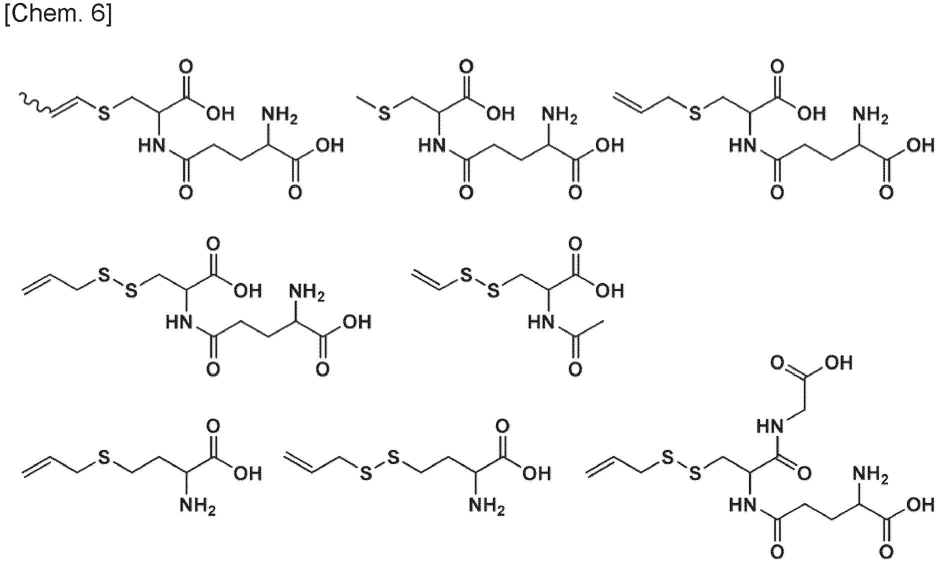

### Effects of the Invention

According to the present invention, it is possible to provide a novel use of a predetermined sulfur-containing compound or a salt thereof, or a composition containing the same.

### BRIEF DESCRIPTION OF THE DRAWINGS

FIG. 1 illustrates sirtuin activation effects of predetermined sulfur-containing compounds;
FIG. 2 illustrates a sirtuin activation action in a cerebral cortex by administrating S-1-propenylcysteine;
FIG. 3 illustrates a change in a SIRT1 protein mass in a hippocampus by administrating S-1-propenylcysteine;
FIG. 4 illustrates changes in NAD⁺ amount in a hippocampus by administrating S-1-propenylcysteine;
FIG. 5 illustrates a change in p53 protein that is a senescent cell marker in a hippocampus by administrating S-1-propenylcysteine;
FIG. 6 illustrates a change in expression level of SIRT1 in a kidney by administrating S-1-propenylcysteine;
FIG. 7 illustrates changes in expression levels of p16 and p21 genes that are senescent cell markers in a kidney by administrating S-1-propenylcysteine;
FIG. 8 illustrates changes in KIM-1 and NGAL gene protein masses that are markers of kidney injuries in a kidney by administrating S-1-propenylcysteine;
FIG. 9 illustrates cognition/memory retention by administrating S-1-propenylcysteine;
FIG. 10 illustrates changes in NAD⁺ amounts in a kidney, a skeletal muscle, and a liver by administrating S-1-propenylcysteine;
FIG. 11 illustrates changes in SIRT1 protein masses in a cerebral cortex, a hippocampus, a heart, a lung, a liver, a kidney, and a skeletal muscle by administrating S-1-propenylcysteine;
FIG. 12 illustrates a change in a SIRT1 protein mass in a hypothalamus by administrating S-1-propenylcysteine; and
FIG. 13 illustrates changes in SIRT1 protein masses in a liver and a heart by administrating S-1-propenylcysteine.

### PREFERRED MODE FOR CARRYING OUT THE INVENTION

### <Agent>

The sulfur-containing compound used in the present invention is a compound (excluding S-1-propenylcysteine) represented by the general formula 1A or 1B or a salt thereof. The sulfur-containing compound or a salt thereof may be used alone or in combination of two or more types thereof. In the latter case, the two or more types of single agents may be used in combination or in the form of a medical compound. 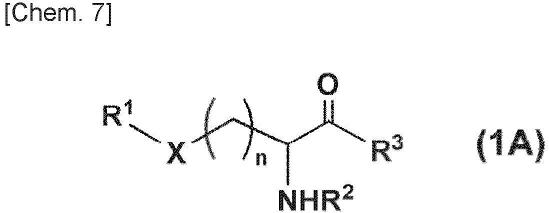 (In the formula, R¹ represents an alkyl group that may have a substituent, an alkenyl group that may have a substituent, or an alkynyl group that may have a substituent. R² represents a hydrogen atom or an acyl group. R³ represents a hydroxy group or a monosubstituted amino group. X represents a group represented by -S-, -S(O)-, -S(O)₂-, -S-S-, -S-S-S-, or -S-S-S-S-. n represents 1 or 2.) 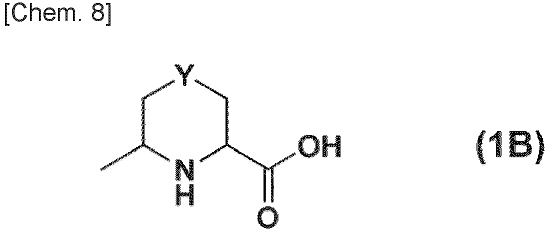 (In the formula, Y represents a group represented by -S- or -S(O)-.)

R¹ may be a C1 to C8 (specifically, any two points between C1 and C8 or one point) alkyl group that may have a substituent, a C2 to C8 (specifically, any two points between C2 and C8 or one point) alkenyl group that may have a substituent, or a C2 to C8 (specifically, any two points between C2 and C8 or one point) alkynyl group that may have a substituent. Examples of the substituent include a hydroxy group, an alkoxy group, an amino group, a carboxy group, an aryl group, a heteroaryl group, etc. More specifically, R¹ may be a C1 to C8 alkyl group (specifically, any two points between C1 and C8 or one point), a hydroxy C1 to C8 alkyl group (specifically, any two points between C2 and C8 or one point), a C2 to C8 alkenyl group (specifically, any two points between C2 and C8 or one point), or a C2 to C8 alkynyl group (specifically, any two points between C2 and C8 or one point).

R² may be a hydrogen atom, a C1 to C3 (specifically, any one or two of C1, C2, or C3) alkylcarbonyl group, or a residue obtained by removing -OH from a carboxy group of an amino acid. Examples of the residue obtained by removing - OH from a carboxy group of an amino acid include a γ-glutamyl group. More specifically, R² may be a hydrogen atom.

R³ may be a hydroxy group or a residue obtained by removing a hydrogen atom from an amino group of an amino acid. Examples of the residue in which one hydrogen atom has been removed from the amino group of an amino acid include, for example, a residue in which one hydrogen atom has been removed from an amino group of glycine. More specifically, R³ may be a hydroxy group.

More specifically, X may be a group represented by -S-, -S(O)-, or -S-S-.

More specifically, Y may be a group represented by -S-.

Examples of specific compounds included in the formulae 1A and 1B are as follows, but are not limited thereto. Although not particularly limited, the compound represented by the formula 1A may be an L isomer, and the compound represented by the formula 1B may be a (3R,5S) isomer.

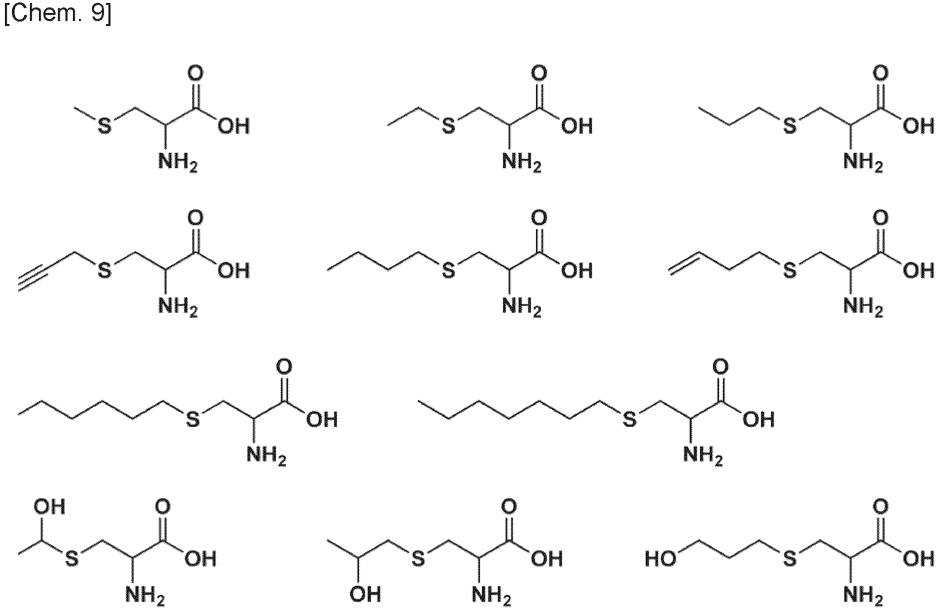

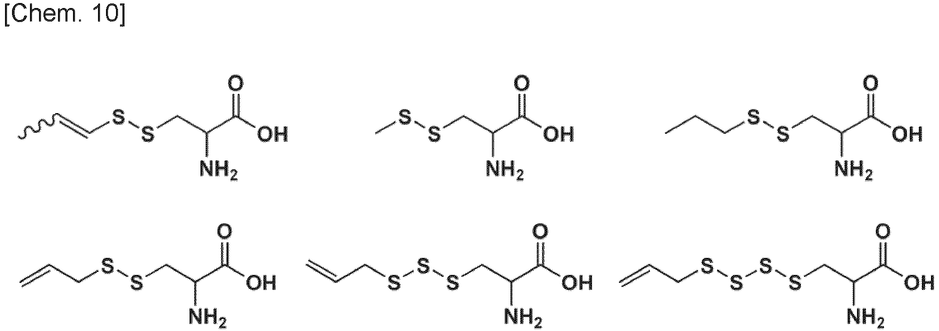

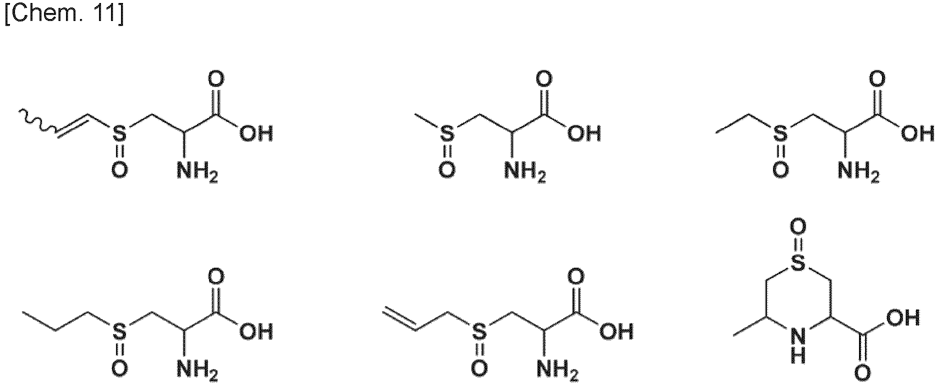

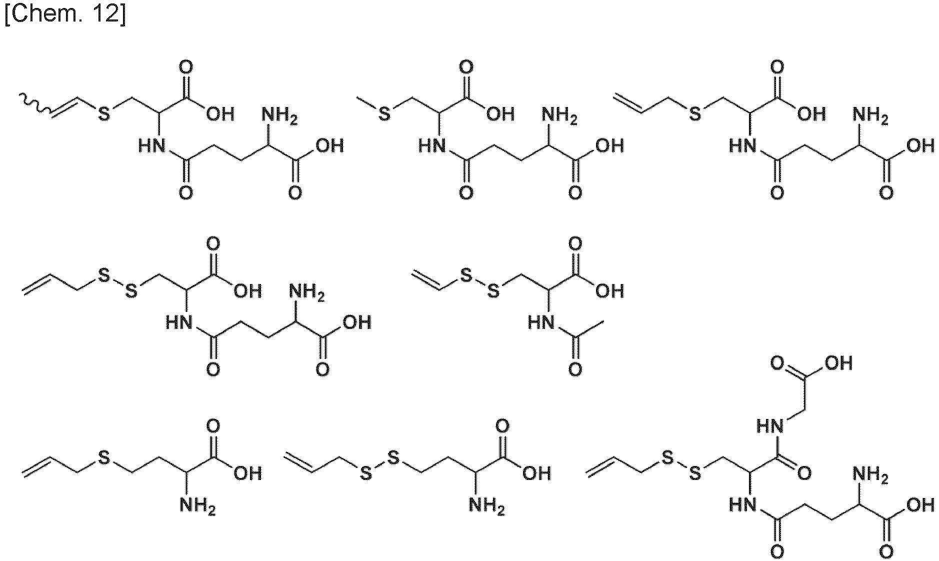

The salt is a physiologically acceptable salt and may be any of an acid addition salt or a base addition salt. Examples of the acid addition salt include (a) salts with mineral acids such as hydrochloric acid, sulfuric acid, nitric acid, or phosphoric acid, (b) salts with organic carboxylic acids such as formic acid, acetic acid, citric acid, fumaric acid, gluconic acid, malic acid, succinic acid, tartaric acid, trichloroacetic acid, or trifluoroacetic acid, and (c) salts with sulfonic acids such as methanesulfonic acid, benzenesulfonic acid, p-toluenesulfonic acid, mesitylenesulfonic acid, or naphthalenesulfonic acid; and examples of the base addition salt include (a) salts with alkali metals such as sodium or potassium, (b) salts with alkaline earth metals such as calcium or magnesium, (c) ammonium salts, and (d) salts with nitrogen-containing organic bases such as trimethylamine, triethylamine, tributylamine, pyridine, N, N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

The sulfur-containing compound or a salt thereof may exist not only in an unsolvated form but also in a hydrate or a solvate, and such a hydrate or solvate may exist in any crystal form depending on manufacturing conditions. Accordingly, the sulfur-containing compound or salt thereof in the present invention encompasses all stereoisomers, hydrates, solvates, and all polymorphic crystalline or amorphous forms.

The sulfur-containing compound or a salt thereof can be obtained by an organic synthesis method. The compound of the formula 1A is not particularly limited, but may be synthesized by, for example, the following two routes.

A compound in which X is a group represented by -S- is obtained by reacting cysteine or homocysteine with R¹Br. Further oxidation gives a compound in which X is a group represented by -S(O)-.

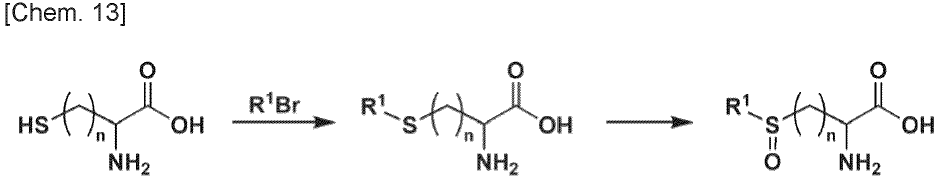

A compound in which X is a group represented by -S-S- is obtained by a thiol exchange reaction between cysteine or homocysteine and R¹-S-S-R¹. Similarly, when reacted with R¹-S-S-S-R¹, a compound in which X is a group represented by -S-S-S- is obtained, and when reacted with R¹-S-S-S-S-R¹, a compound in which X is a group represented by -S-S-S-S- is obtained.

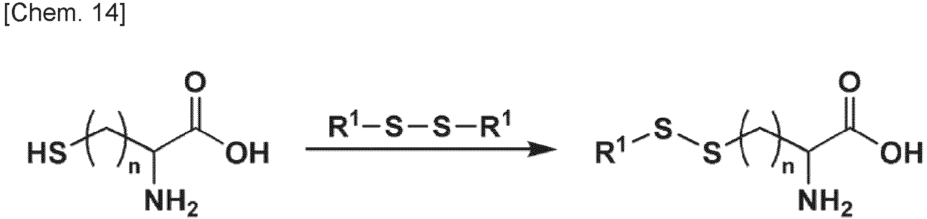

In order to obtain a compound in which R² is an acyl group, the compound obtained above may be reacted with RCOOH or an anhydride thereof. In order to obtain a compound in which R³ is a monosubstituted amino group, the compound obtained above may be reacted with RNH₂.

Although not particularly limited, the compound of the formula 1B can be obtained by known synthetic method (see, for example, Chem. Pharm. Bull., 1981, 29(6), pp. 1554-1560).

When the sulfur-containing compound or a salt thereof naturally occurs, it can also be obtained by processing source thereof (typically an allium genus plant) by various methods. Such a sulfur-containing compound or a salt thereof is preferable on account of being generally low toxic because the source (typically, plants belonging to the genus Allium) is commonly used as a food.

Here, examples of the plant of genus Allium include garlic (Allium sativum L.), onion (Allium cepa L.), elephant garlic (Allium ampeloprasum L.), Allium tuberosum, and green onion (Allium fistulosum L.). Such plants may be used solely or in combination. In addition, as the plant of genus Allium, a raw plant can be used as it is, or if necessary, be removed of its outer shell, cut or shredded, or made into powder or extract using a solvent capable of producing a medicine or food. Examples of the solvent include water and alcohol, and a solvent obtained by adding an acid or a basic substance to the solvent.

As the sulfur-containing compound or a salt thereof, not only an isolated and purified product but also a crude product, plants described above or a processed product thereof, and a fraction in which the content of the sulfur-containing compound or a salt thereof is increased by an extraction operation from the plants described above can be used.

As shown in the Test Examples below, various sulfur-containing compounds satisfying the formulae 1A and 1B increased SIRT1 activity in human cells. In addition, SIRT1 activity was increased in the cerebral cortex of senescence-accelerated mice, and SIRT1 expression was increased in the hippocampus, kidney, hypothalamus, heart, lung, liver, and skeletal muscle. Therefore, the sulfur-containing compound, the salt thereof, or the composition containing them can be an agent for activating or enhancing expression of a sirtuin or Klotho. Further, NAD⁺ (which is known to have a function of improving the function of sirtuins) was also increased in the brain, kidney, skeletal muscle and liver of animals. Therefore, the sulfur-containing compound, the salt thereof, or the composition containing them can be a NAD⁺ increasing agent. In addition, cells that express p16 and p21 as markers of senescent cells in the kidney and p53 in the hippocampus were suppressed. Therefore, the sulfur-containing compound, the salt thereof, or the composition containing them can be a senescent cell suppressor.

Given common technical knowledge that activation or expression enhancement of a sirtuin, senescent cell suppression, and NAD⁺ increase have mutually common mechanisms, the sulfur-containing compound, the salt thereof, or the composition containing them can be used as a sirtuin activation or expression enhancement agent, an NAD⁺ increase agent, or a senescent cell suppression agent, each of which are for acting in the brain, kidney, skeletal muscle, liver, and heart.

The sirtuin activation or expression enhancer and the senescent cell inhibitor of the present invention may be used for preventing, treating or ameliorating symptoms resulting from low activity or low expression of sirtuins, or symptoms resulting from an increase in senescent cells. As described above, functional deterioration of SIRT1 and accelerated cellular senescence are considered to be related to symptoms and diseases such as kidney injuries, cognitive/memory disorders (including short-term and long-term), liver steatosis, cardiac dysfunction, and fatty liver (Non-Patent Documents 2 to 4, 9, and 10). Examples of the kidney injuries include diseases with highly-expressed KIM-1 and NGAL, acute kidney injury, diabetic nephropathy, and nephrotic syndrome. Examples of the cognitive/memory disorders include neurological disorders in cerebral cortex or the hippocampus, learning disorders, and memory disorders (including short-term and long-term).

In the present invention, "sirtuins" mean sirtuin proteins and homologs thereof. Humans are known to have 1 to 7 sirtuins (SIRT1 to SIRT7). However, SIRT1 is preferred in the present invention.

The "sirtuin expression enhancement" means an increase in a transcript of a sirtuin gene, and/or a sirtuin protein, and includes, for example, activating transcription and/or translation of a gene, improving stability of the transcript and/or the protein, inhibiting degradation of the transcript and/or proteolysis, etc.

In the present invention, the "cellular senescence" refers to a phenomenon in which the irreversible cell cycle arrest occurs in a cell. The cellular senescence has been observed, for example, in brain cells (such as hippocampal cells and cerebral cortex cells), kidney cells, liver, and skeletal muscle. The "senescent cell suppression" refers to one or both of removal of senescent cells and prevention or delay of a cellular senescence phenomenon.

The p16, p21, and p53 genes are known as molecular markers of DNA damage that causes the cellular senescence. However, the senescent cell inhibitor of the present invention more preferably suppresses mitochondrial dysfunction that accelerates the cellular senescence and DNA damage. In addition, the senescent cells can be more effectively suppressed by enhancing the immune functions.

The NAD⁺ increasing agent of the present invention may be used for preventing, treating, or ameliorating symptoms resulting from a low NAD⁺ level. As described above, low presence of NAD⁺ has been implicated to be associated with symptoms and diseases such as nonalcoholic fatty liver disease, kidney disorder, etc. (Non-Patent Documents 2 to 4 and 9 to 10). Examples of kidney injuries include diseases with highly-expressed KIM-1 and NGAL, acute kidney injury, diabetic nephropathy, and nephrotic syndrome.

The NAD⁺ increase agent, sirtuin-activating/expression-enhancing agent, and senescent cell suppressor of the present invention may be in the form of a pharmaceutical drug or a food, or may be in the form of a material or a preparation to be added thereto.

In addition, the food includes food whose concept is NAD⁺ increase, sirtuin activation/expression enhancement, or senescent cell suppression and which explains and displays an action on the basis of their function as necessary, functional food, food with a functional claim, food for the sick, food for specified health uses, and nutritional supplements (supplements).

A dosage form of the medicine is preferably a dosage form suitable for oral administration. As a specific dosage form of an oral administration preparation, for example, a solid preparation may be in a form of a tablet, a capsule, a fine particle, a pill, or a granule, and a liquid preparation may be in a form of an emulsion, a solution, a suspension, or a syrup. Such a pharmaceutical preparation can be prepared according to an ordinary method by appropriately blending an excipient, a binder, a disintegrating agent, a lubricant, a coloring agent, a flavoring agent, a pH adjusting agent or the like with the sulfur-containing compound of the present invention or a salt thereof as necessary. However, the dosage form of the medicine is not particularly limited, and may be a dosage form suitable for parenteral administration, for example, a dosage form suitable for intravenous (an injection and catheter), transmucosal (a liquid or ointment), or intracranial administration (a catheter).

A form of S-1-propenylcysteine food is not particularly limited, and can take various forms such as a solid food product, a semi-fluid food product, a gel-like food product, a tablet, a caplet, and a capsule, and more specifically a form of various food products such as a confection, a beverage, a seasoning, a processed fishery product, a processed meat food product, bread, and a health food product. Such food can be produced by a conventional method by appropriately blending the sulfur-containing compound of the present invention or a salt thereof with a food material which is generally used in the production of these foods.

The pharmaceutical drug or food may contain other substances such as resveratrol and nicotinamide mononucleotide, which are involved in increasing NAD⁺, activation or expression enhancement of a sirtuin and senescent cell suppression. In addition, for example, vitamins, lipids, and minerals that alleviate inflammation, such as vitamin C, vitamin E, vitamin B2, vitamin B6, niacin, hesperidin, α-lipoic acid, glutathione, coenzyme Q10, zinc, magnesium, and omega-3 fatty acid can be contained.

A preferred ingestion amount of the aforementioned pharmaceutical drug or food per day varies depending on factors, for example, subjects who ingest, forms of ingestion, types of materials, additives to be ingested simultaneously, and ingestion intervals. However, it is preferable to ingest 0.001 to 10 mg/kg of the sulfur-containing compound or a salt thereof per day, and it is more preferable to ingest 0.1 to 1 mg/kg per day. In addition, a daily dose can be optionally divided into 2 to 4 times for ingestion. The amount of the sulfur-containing compound or a salt thereof may be an amount of one type or a total amount of two or more types.

Subjects to be administered or subjects that ingest may be organisms with decreased activity or decreased expression of surtuins, increased senescent cells, or decreased amounts of NAD⁺, and may be, however, a healthy organism without a particular disease (e.g., diabetes mellitus or renal injury). The organisms are not limited to the animals described so far, and include plants, fungi (including yeast), nematodes, and cells (which may be derived from the above-mentioned animals or plants), and animals are preferred. Examples of the animals include vertebrates (preferably rodents and primates), fish, birds, insects, and reptiles, and in particular, rodents (particularly rats and mice) and primates (particularly humans and monkeys) are preferable.

To the best of our knowledge, there is no proven fact that administration of the sulfur-containing compounds of the formulae 1A and 1B to animals activated sirtuins or enhanced expression of sirtuins, increased NAD⁺ in the bodies of the animals, or senescence of non-cancerous cells in the animals were suppressed. These facts were unexpectedly discovered as a result of intensive studies by the present inventors.

Examples of the living organisms include humans suffering from e.g., kidney injuries, cognitive/memory disorders (including short and long terms), liver steatosis, cardiac dysfunction, fatty liver, and nonalcoholic fatty liver disease, or animals (for example, humans) hoping to prevent the diseases. However, healthy living organisms are also preferable.

Plant sirtuins have been suggested to be involved in genome instability, protection from cellular oxidative damage, gamete formation, fruit development and maturation, leaf aging, and regulation of photosynthetic activity (Front Plant Sci. 2018, Jul 5; 9: 961). The plant includes, but is not particularly limited to, e.g., eggplants such as tomato, bell pepper, pepper, eggplant, etc.; gourds such as cucumbers, squash, melon, watermelon, etc.; green vegetables/condiment vegetables such as celery, parsley, lettuce, etc.; green onions such as green onion, onion, garlic, etc.; beans such as soybean, peanut, haricot bean, pea, adzuki bean, etc.; other fruit vegetables such as strawberry, etc.; straight roots such as radish, turnip, carrot, burdock root, etc.; potatoes such as taro, cassava, potato, sweet potato, Chinese yam, etc.; soft leaf vegetables such as asparagus, spinach, Japanese honewort, etc.; flowers such as prairie gentian, stock, carnation, chrysanthemum, etc.; grains such as rice, corn, etc.; lawns such as bentgrass, korai lawn grass, etc.; oil crops such as rapeseed, sunflower, etc.; sugar crops such as sugarcane, sugar beet, etc.; fiber crops such as cotton, rush, etc.; forage crops such as clover, sorghum, dent corn, etc.; deciduous fruit trees such as apple, pear, grape, peach, etc.; citrus such as mandarin orange, lemon, grapefruit, etc.; and trees such as satsuki azalea, azalea, cedar, etc. Dosage forms for administration to plants include powders, granulated powders, granules, wettable powders, flowable agents, emulsions, pastes, etc. The sulfur-containing compound or the salt thereof may be administered directly or indirectly through soil, a hydroponic solution or a medium. Specific examples include soil treatment agents, foliage treatment agents, seed treatment agents before sowing, treatment agents for plants before transplantation, treatment agents for plants during transplantation, hydroponic solutions, culture media, and etc.

### <Another Embodiment>

In another embodiment, the present invention may relate to a compound represented by the general formula 1A or 1B (excluding S-1-propenylcysteine) or a salt thereof for use in various applications described above, or a composition containing a compound represented by the general formula 1A or 1B (excluding S-1-propenylcysteine) or a salt thereof for use in various applications described above. In another embodiment, the present invention may relate to a method of administering a compound represented by the general formula 1A or 1B (excluding S-1-propenylcysteine) or a salt thereof, or a composition containing a compound represented by the general formula 1A or 1B (excluding S-1-propenylcysteine) or a salt thereof, for use in the various uses described above. In another embodiment, the present invention may relate to use of a compound represented by the general formula 1A or 1B (excluding S-1-propenylcysteine) or a salt thereof or a composition containing the compound represented by the general formula 1A or 1B (excluding S-1-propenylcysteine) or a salt thereof as various applications described above. In another embodiment, the present invention may relate to use of the compound represented by the general formula 1A or 1B (excluding S-1-propenylcysteine) or a salt thereof or the composition containing the compound represented by the general formula 1A or 1B (excluding S-1-propenylcysteine) or a salt thereof in the manufacture of a pharmaceutical drug for use in various applications described above. The details of these embodiments are similar to those of the agents described above.

### EXAMPLES

### Reference Synthesis Example (S-1-propenylcysteine)

t-potassium butoxide was dissolved in dimethylsulfoxide, S-allylcysteine was added, and the resulting mixture was stirred for 10 minutes. A reaction container containing the resulting mixture was transferred to an ice bath and a 12 M hydrochloric acid solution was added while being vigorously stirred. Ethanol was then added, and the resulting crystals were collected by filtration. The obtained crystal was recrystallized with 30% ethanol to obtain S-1-propenylcysteine.

### Synthesis Example 1 (S-propylcysteine: SPC)

Cysteine was dissolved in water, and an ethanol solution of propyl bromide was added and stirred. Triethylamine was added dropwise to the mixed solution, and the resulting mixture was stirred overnight at room temperature. After concentration, ethanol was added, and the precipitated crystals were collected by filtration. The crystals were washed with ethanol and then recrystallized from an aqueous solvent to obtain S-propylcysteine.

### Synthesis Example 2 (5- methylthiomorpholine-3-carboxylic acid: Cyclo-SAC)

5-methylthiomorpholine-3-carboxylic acid was obtained according to the method described in Chem. Pharm. Bull. 1981; 29(6), pp. 1554-1560.

### Synthesis Example 3 (S-3-butenylcysteine: SBenC)

S-3-butenylcysteine was obtained according to the method of Synthesis Example 1 except that propyl bromide was changed to butenyl bromide.

### Synthesis Example 4 (S-2-hydroxypropylcysteine: S2HyPC)

Cysteine was dissolved in a mixed solvent of methanol and water and stirred. Under an ice bath, a 28% aqueous ammonia solution was added, and a methanol solution of 1-bromo-2-propanol was added dropwise. After stirring for 10 minutes, the mixture was returned to room temperature and stirred overnight. The reaction mixture was subjected to liquid-liquid extraction with chloroform, and the aqueous layer was concentrated. The crystals obtained after concentration were purified using HPLC to give S-2-hydroxypropylcysteine.

### Synthesis Example 5 (S-methylmercaptocysteine: SMMC)

Dimethyl disulfide and hydrogen peroxide were added to acetic acid, and the resulting mixture was stirred. Cysteine was then added and stirred. The mixture was neutralized with an aqueous ammonia solution, and the precipitated crystals were washed with water and methanol. The crystals were dissolved in a 1 N hydrochloric acid solution and washed with diethyl ether. Thereafter, aqueous ammonia solution was added for neutralization, and the precipitated crystals were washed with water and methanol to obtain S-methylmercaptocysteine.

### Synthesis Example 6 (Methiin)

Methyl bromide was added to a 2 N aqueous ammonia solution, and the resulting mixture was stirred. Cysteine was then added and further stirred. The obtained solution was concentrated, and the resulting crystals were collected. The obtained crystals were washed with water and ethanol to obtain S-methylcysteine. This S-methylcysteine was oxidized with hydrogen peroxide, and the obtained crystal was recrystallized with a mixed solvent of acetone and water. The precipitated crystal was washed with acetone to obtain methiin.

### Synthesis Example 7 (Ethiin)

Ethyl bromide was added to a 2 N ammonia solution, and the resulting mixture was stirred. Cysteine was then added and further stirred. The obtained solution was concentrated, and the resulting crystals were collected. The obtained crystals were washed with water and ethanol to obtain S-ethylcysteine. This S-ethylcysteine was oxidized with hydrogen peroxide, and the obtained crystal was recrystallized with acetone and water. The precipitated crystals were washed with acetone to obtain ethiin.

### Synthesis Example 8 (Alliin)

Allyl bromide was added to a 2 N aqueous ammonia solution, and the resulting mixture was stirred. Cysteine was then added and further stirred. The solution was concentrated, and the resulting crystals were collected. The obtained crystals were washed with water and ethanol to obtain S-allylcysteine. This S-allylcysteine was oxidized with hydrogen peroxide, and the obtained crystal was recrystallized with acetone and water. The precipitated crystal was washed with acetone to obtain alliin.

### Test Examples

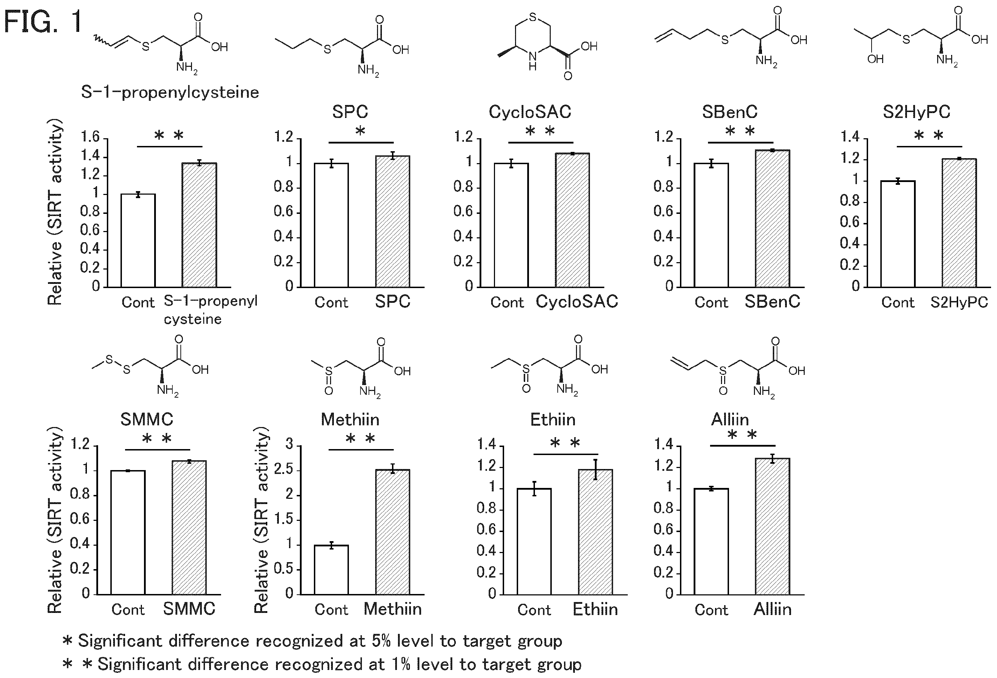

To 200 ng of a cell lysate of human uterocervical cancer cells, trichostatin A and dithiothreitol were added at concentrations of 50 µM and 10 mM, respectively and the mixture was left to stand at 37°C for 30 minutes to promote the degradation of endogenous NAD⁺. Next, each of the sulfur-containing compounds synthesized above was added at a concentration of 3 mM, and the mixture was left to stand at 37°C for 10 minutes, followed by enzymatic reaction using Cyclex SIRT1/Sir2 Deacetylase Fluorometric Assay Kit Ver. 2 (manufactured by Medical & Biology Laboratories Co., Ltd.) to measure sirtuin activity. The results are shown in FIG. 1. All of the sulfur-containing compounds increased sirtuin activity (FIG. 1).

### Preparation Example 1 Ethanol-Extracted Fraction of Garlic

About 1 kg of peeled garlic bulbs and about 1000 mL of 30% ethanol were put in a container and sealed. The container was left at room temperature for 1 to 10 months, and stirred as appropriate. Solid and liquid were separated from the mixture, and the liquid was dried by spray-drying to obtain a yellow-brown powder.

### Preparation Example 2 Isolation of S-1-Propenylcysteine from the Ethanol-Extracted Fraction of Garlic

(1) The ethanol-extracted fraction of garlic obtained in Preparation Example 1 was put into a dialysis tube with a pore size of 3500, and dialyzed against purified water. The dialysate was passed through a cation exchange resin Dowex 50Wx8 (H+), and the resin was thoroughly washed with purified water. Amino acids adsorbed to the resin were eluted with 2 N ammonia and concentrated under reduced pressure. A concentrate was applied to a silica gel column, and subjected to column chromatography using a chloroform/methanol/water mixture as a solvent. The fraction containing the target product (S-1-propenylcysteine) were collected and concentrated. The concentrate was dissolved in water, and chromatography was performed using a preparative reversed-phase column (the octadecylsilyl column) with 0.1% formic acid as a solvent to collect the target product, and the solvent was removed by freeze drying. The obtained lyophilizate was confirmed to be a mixture of trans-S-1-propenylcysteine and cis-S-1-propenylcysteine (trans isomer: cis isomer=8: 2) by comparing the structure with spectra obtained from the following standard substances using NMR (solvent: heavy water) and a mass spectrometer.

### Trans-S-1-propenylcysteine

¹H-NMR (500 MHZ, in D₂O-NaOD, δ): 1.76 (d, 3H, J=7.0 Hz), 2.98 (dd, 1H, J=7.5, 14.5 Hz), 3.14 (dd, 1H, J=4.5, 14.5 Hz) 3.69 (dd, 1H, J=4.5, 7.5 Hz), 5.10-5.14 (m, 1H), 6.02 (d, 1H, J=15.5 Hz);
¹³C-NMR (125 MHz, in D₂O-NaOD, δ): 17.61, 33.53, 53.70, 119.92, 132.12, 172.73,
HRMS: observed [M+H]⁺=162.0583, calculated [M+H]⁺=162.0581.

### Cis-S-1-propenylcysteine

¹H-NMR (500 MHz, in D₂O, δ): 1.74 (d, 3H, J=7.0 Hz), 3.21 (dd, 1H, J=7.5, 15.0 Hz), 3.31 (dd, 1H, J=4.5, 15.0 Hz), 3.95 (dd, 1H, J=4.5, 7.5 Hz), 5.82-5.86 (m, 1H), 6.01 (d, 1H, J=9.5 Hz);
¹³C-NMR (125 MHz, in D₂O-NaOD, δ): 13.89, 33.88, 54.16, 122.58, 127.78, 172.63.
HRMS: observed [M+H]⁺=162.0580, calculated [M+H]⁺=162.0581.

### (2) Measurement of S-1-Propenylcysteine in the Ethanol-Extracted Fraction of Garlic

500 mg to 1 g of the ethanol-extracted fraction of garlic obtained in Preparation Example 1 (1) was put in a container, and 20 mM hydrochloric acid solution of S-n-3 butenyl cysteine was added thereto as an internal standard, and the volume was adjusted to 20 mL with 20 mM hydrochloric acid. After the mixture was well stirred, a part thereof was taken and centrifuged at 1750 G for about 10 minutes. A part of the supernatant obtained was taken and subjected to centrifugal filtration using a centrifugal filtration unit (Amicon Ultra, cutoff: 3000) (15000 rpm, 10 min). 20 µL of the obtained filtered product was taken and derivatized using an AccQ-Tag Derivatization Kit (Waters). Separately, a standard compound was dissolved in 20 mM hydrochloric acid, and the same procedure as for a sample was performed to prepare a standard solution for calibration curve. A sample solution and the standard solution were chromatographed on an Acquity UPLC system (Waters) to determine the content. As a result, S-1-propenylcysteine was of 3.7±0.3 mg/g dry matter.

### Reference Test Example

### Sirtuin activation action

### (1) Sample preparation

Preparation of a test liquid for evaluating biological activity was performed as follows. When evaluating the biological activity, all test liquids were prepared at the time of use.
(a) About 5 mg of S-1-propenylcysteine (cis/trans mixture) produced in Preparation Example 2 was weighed precisely, and dissolved in 10 mL of purified water to prepare an administration liquid.
(b) About 6 mg of S-1-propenylcysteine (cis/trans mixture) produced in Preparation Example 2 was weighed precisely and dissolved in 1 mL of culture liquid. The liquid was used as a stock solution, diluted appropriately, and subjected to an in vitro test.

### (2) Animals for Evaluation Tests

Animals for evaluating the biological activity were bred as follows. Senescence-accelerated mice SAMP8 (male) for testing were purchased from Japan SLC, Inc. After purchase, the animals were acclimatized for one week and used as animals for the evaluation tests.

### (3) Preparation of Human Neuroblasts for Evaluation Tests

Human neuroblast line SH-SY5Y cells were cultured in Dulbecco's Modified Eagle's Medium added with 10% fetal bovine serum, antibiotic penicillin, and streptomycin solution, and were used as cells for the evaluation tests.

### (4) Sirtuin Activation Test

A SIRT1_GLO kit manufactured by Promega Corporation was purchased to measure the sirtuins activity. A lysate of the cerebral cortex was placed in a 96-well plate, various reaction reagents were added, and then luminous intensity of luciferase was measured by a plate reader.

### (a) SIRT1 Activation Action (In Vivo):

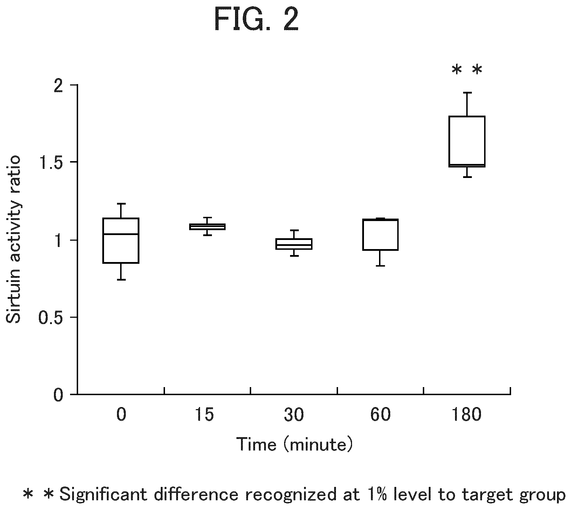

A single dose of the sample prepared in the above (1) (a) was orally administrated to animals for the evaluation tests in the above (2), and then cerebral cortices of such animals were extracted 15, 30, 60, and 180 minutes later. The cerebral cortices were crushed using an automatic tissue crusher and lysed by adding a cell dissolution liquid. Centrifugation was performed and the sirtuins activity in the supernatant thereof was measured. The results are shown in FIG. 2. Sirtuin activity increased 180 minutes after administration of S-1-propenylcysteine.

### (Figure 2)

### (b) SIRT1 Protein Increasing Action (In Vivo):

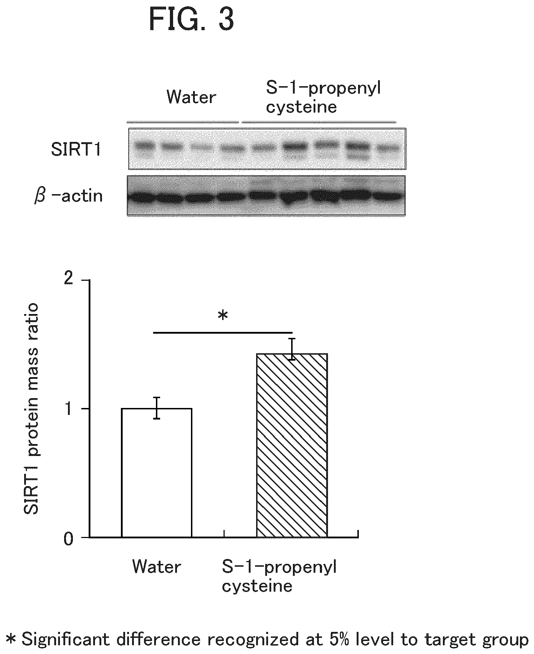

After repeated oral administration of the sample prepared in the above (1) (a) to animals for the evaluation tests in the above (2) for two weeks, hippocampi of such animals were excised. After the hippocampi were crushed using an automatic tissue crusher, the cells were lysed with a RIPA cell lysis buffer manufactured by Millipore Corporation which was diluted 10-fold with purified water added with combined protease/phosphatase inhibitors manufactured by Thermo Fisher Scientific K.K., and then centrifuged (10,000 rpm, 10 min, 4° C.), and the supernatant was used as a cell extract. The cell extract was used for analysis by Western Blotting according to a conventional method. An anti-SIRT1 antibody (manufactured by Biolegend) and an anti-β-actin antibody (manufactured by Fujifilm Wako Pure Chemical Corporation) were used as antibodies. The results are shown in FIG. 3. S-1-propenylcysteine increased the SIRT1 protein mass.

### (C) NAD⁺ Amount Increasing Action (In Vivo):

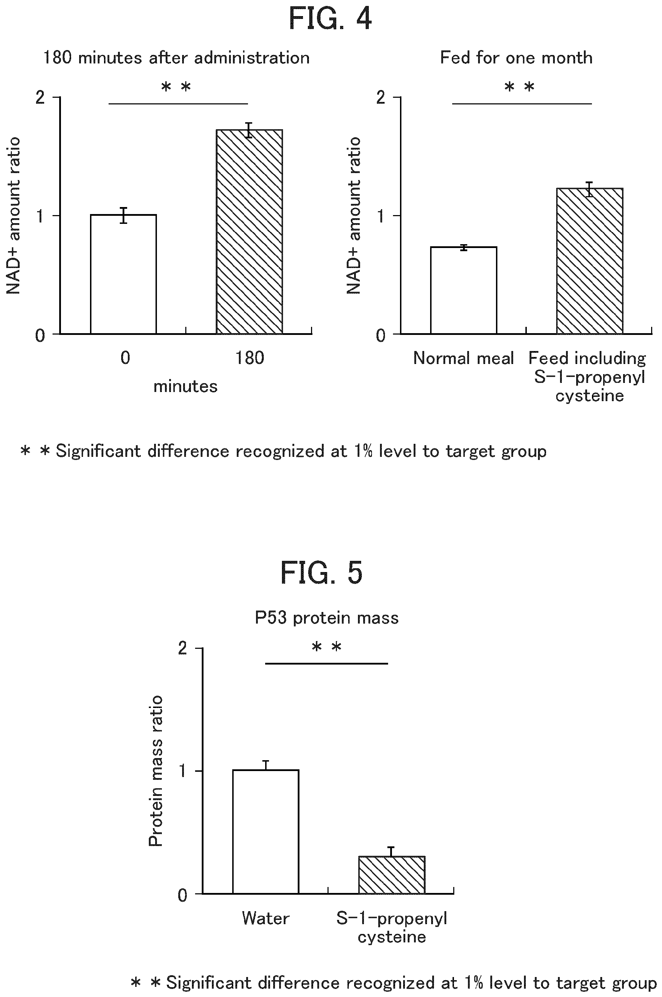

A single dose of the sample prepared in (1) (a) was orally administered to animals for the evaluation tests in the above (2), and then cerebral cortices of such animals were excised 180 minutes later. In addition, the sample prepared in the above (1) (a) was mixed with food and fed to animals for the evaluation tests in the above (2) for one month, and then cerebral cortices of such animals were excised. The NAD⁺ amount in the cerebral cortices was measured using NAD⁺/NADH Assay Kit manufactured by Dojindo Laboratories. The results are shown in FIG. 4. S-1-propenylcysteine increased NAD⁺ amount.

### (5) Suppression Action on Expression of Cellular Senescence Marker p53 Protein in the Hippocampus (In Vivo):

After repeated oral administration of the sample prepared in the above (1) (a) to animals for the evaluation tests in the above (2) for six weeks, hippocampi of such animals were excised. After the hippocampi were crushed using an automatic tissue crusher, the cells were lysed with a RIPA cell lysis buffer manufactured by Millipore Corporation which was diluted 10-fold with purified water added with a protease inhibitor and a phosphatase inhibitor manufactured by Roche, and then centrifuged (10,000 rpm, 10 min, 4° C.), and the supernatant was used as a cell extract. The cell extract was used for analysis by Western Blotting according to a conventional method. An anti-p53 antibody (manufactured by Proteintech Group, Inc.) and an anti-β-actin antibody (manufactured by Medical and Biological Laboratories Co., Ltd.) were used as antibodies. The results are shown in FIG. 5. S-1-propenylcysteine reduced a p53 protein mass.

### (6) Enhancement action of SIRT1 gene expression in kidney (in vivo):

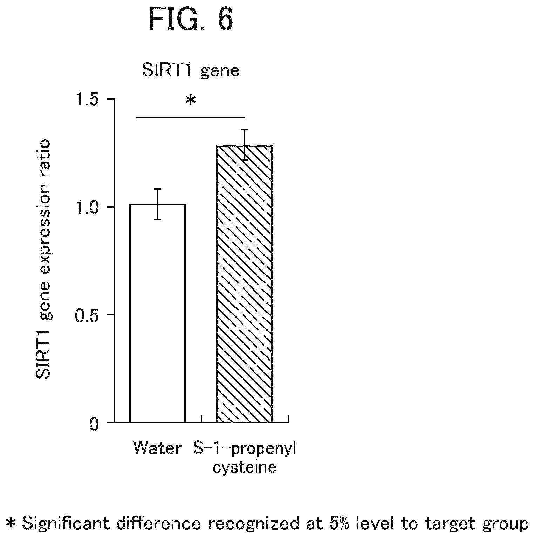

After repeated oral administration of the sample prepared in the above (1) (a) to the animals for the evaluation tests in the above (2) for two weeks, kidneys of such animals were excised. The kidneys were crushed using an automatic tissue crusher, and then RNA was extracted using a TRIzol solution manufactured by Thermo Fisher Scientific K.K. cDNA was synthesized using PRIMEScript RT reagent Kit with gEraser manufactured by Takara. The synthesized cDNA was subjected to real-time PCR using KAPA SYBR Fast qPCR kit manufactured by Nippon Genetics Co., Ltd. The results are shown in FIG.6. S-1-propenylcysteine increased the SIRT1 expression in the mouse kidney.

### (7) Suppression Action on Expression of Senescent Cell Markers p16 and p21 Genes in the Kidney (In Vivo):

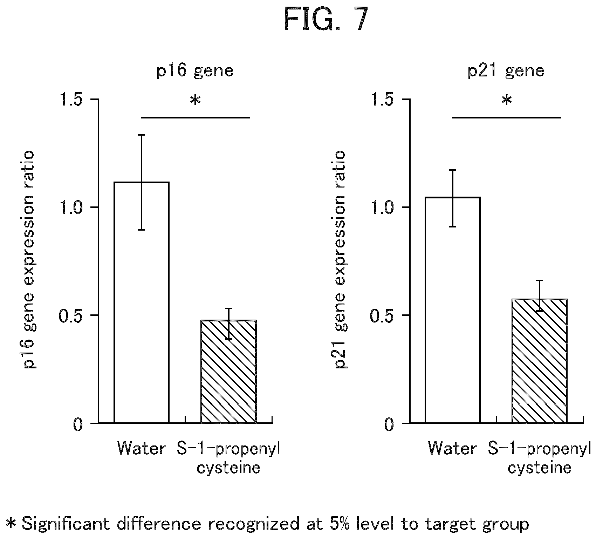

After repeated oral administration of the sample prepared in the above (1) (a) to the animals for the evaluation tests in the above (2) for two weeks, kidneys of such animals were excised. The kidneys were crushed using an automatic tissue crusher, and then RNA was extracted using a TRIzol solution manufactured by Thermo Fisher Scientific K.K. cDNA was synthesized using PRIMEScript RT reagent Kit with gEraser manufactured by Takara. The synthesized cDNA was subjected to real-time PCR using KAPA SYBR Fast qPCR kit manufactured by Nippon Genetics Co., Ltd. The results are shown in FIG. 7. S-1-propenylcysteine reduced cells expressing p16 and p21 genes in the kidneys of senescence-accelerated mice.

### (8) Inhibitory Action on Kidney Injuries (In Vivo)

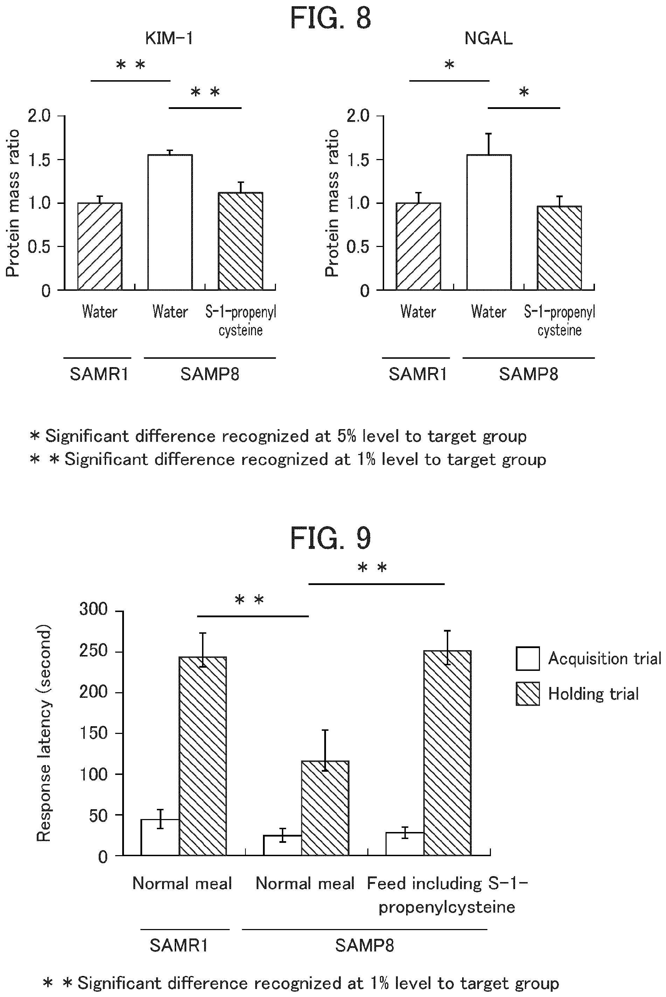

After repeated oral administration of the sample prepared in the above (1) (a) to the animals for the evaluation tests in the above (2) for two weeks, kidneys of such animals were excised. After the kidneys were crushed using an automatic tissue crusher, the cells were lysed with an RIPA cell lysis buffer manufactured by Millipore Corporation which was diluted 10-fold with purified water added with a protease inhibitor and a phosphatase inhibitor manufactured by Roche and then centrifuged (10,000 rpm, 10 min, 4° C.), and the supernatant was used as a cell extract. The cell extract was used for analysis by Western Blotting according to a conventional method. A kidney injury molecule (KIM-1) antibody (manufactured by R&D System) and a lipocalin-2 (NGAL) antibody (manufactured by Proteintech Group, Inc.), KIM-1 and NGAL being markers of kidney injuries, and an anti-GAPDH antibody (manufactured by Fujifilm Wako Pure Chemical Corporation) were used as antibodies. The results are shown in FIG. 8. S-1-propenylcysteine reduced KIM-1 and NGAL.

### (9) Cognitive/Memory Retention Action (In Vivo):

The sample prepared in the above (1) (a) was mixed with food and fed to animals for evaluation test in the above (2) for four months, and then a passive avoidance test was performed. The passive avoidance test is a test utilizing a habit that a mouse prefers a dark place, and on Day 1, a mouse already in a bright room was moved to a dark room while receiving an electrical shock (50 V/1 second), and the time it took for the mouse to enter the dark room was taken as response latency of an acquisition trial. After two months, the mouse was put in the bright room again and the time it took to move to the dark room (the response latency of a holding trial) was used as an index of memory. The results are shown in FIG. 9. S-1-propenylcysteine ameliorated aging-related decline in cognitive and memory functions.

### (10) NAD⁺ amount increasing action in kidney, skeletal muscle, and liver (in vivo):

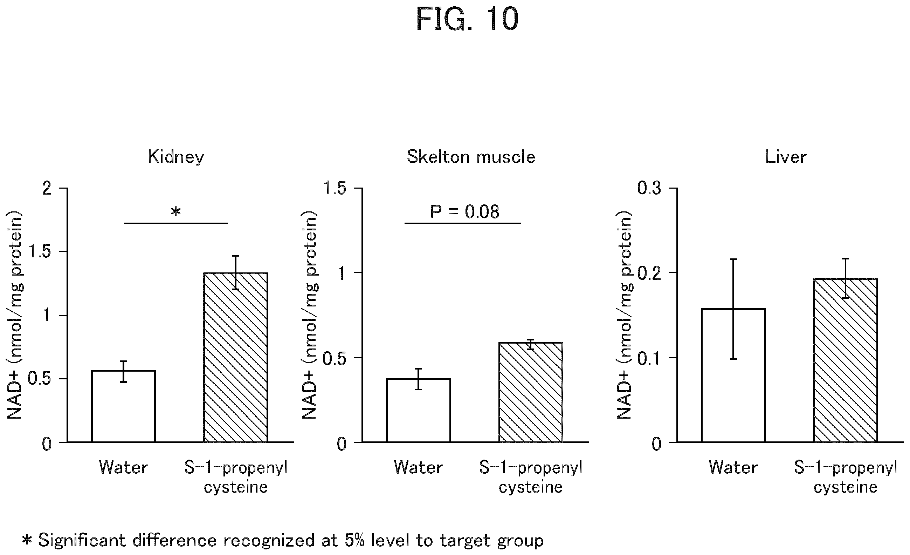

A single dose of the sample prepared in the above (1)(a) was orally administrated to animals for the evaluation tests in the above (2), and then cerebral cortexes, livers, kidneys, and skeletal muscles of such animals were extracted 60 minutes later. The NAD⁺ amounts in the cerebral cortexes, livers, kidneys, and skeletal muscles were measured using NAD⁺/NADH Assay Kit manufactured by Dojindo Laboratories. The results are shown in FIG. 10. S-1-propenylcysteine increased the NAD⁺ amount.

(11) SIRT1 protein increasing action in cerebral cortex, hippocampus, heart, lung, liver, kidney, and skeletal muscle (in vivo):

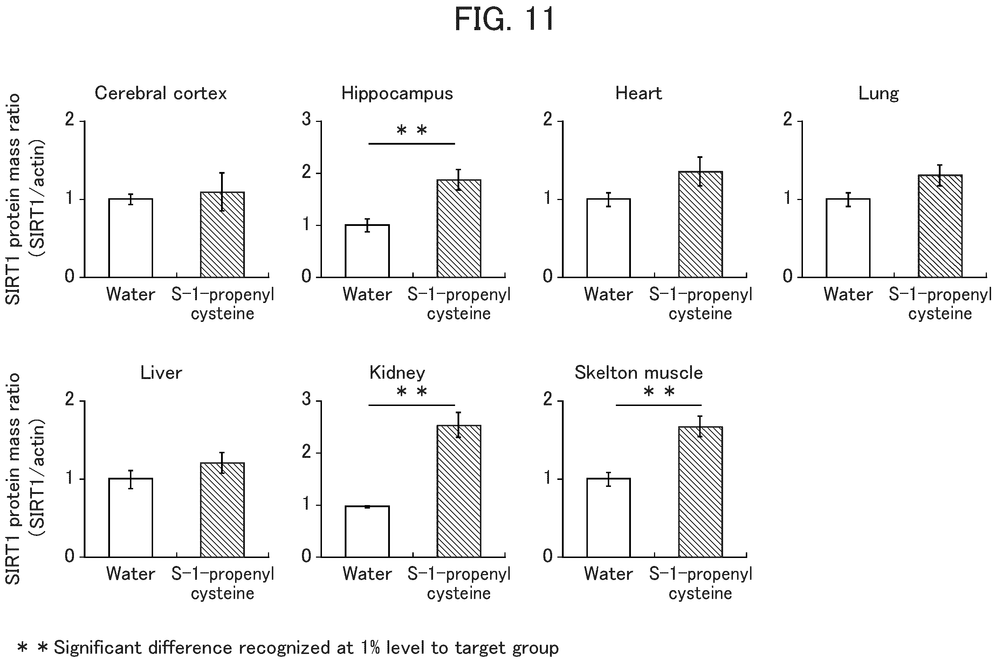

About 10 mg of S-1-propenylcysteine (cis/trans mixture) produced in Preparation Example 2 was weighed precisely, and dissolved in 10 mL of purified water to prepare an administration liquid. After repeated oral administration of the prepared sample to animals for the evaluation tests in the above (2) for two weeks, cerebral cortexes, hippocampi, hearts, lungs, livers, kidneys, and skeletal muscles of such animals were excised. After the cerebral cortexes, hippocampi, hearts, lungs, livers, kidneys, and skeletal muscles were crushed using an automatic tissue crusher, the cells were lysed with a RIPA cell lysis buffer manufactured by Millipore Corporation which was diluted 10-fold with purified water added with combined protease/phosphatase inhibitors manufactured by Thermo Fisher Scientific K.K., and then centrifuged (10,000 rpm, 10 min, 4°C), and the supernatant was used as a cell extract. The cell extract was used for analysis by Western Blotting according to a conventional method. An anti-SIRT1 antibody (manufactured by Biolegend) and an anti-β-actin antibody (manufactured by Fujifilm Wako Pure Chemical Corporation) were used as antibodies. The results are shown in FIG. 11. S-1-propenylcysteine increased the SIRT1 protein mass.

### (12) SIRT1 protein increasing action in hypothalamus (in vivo):

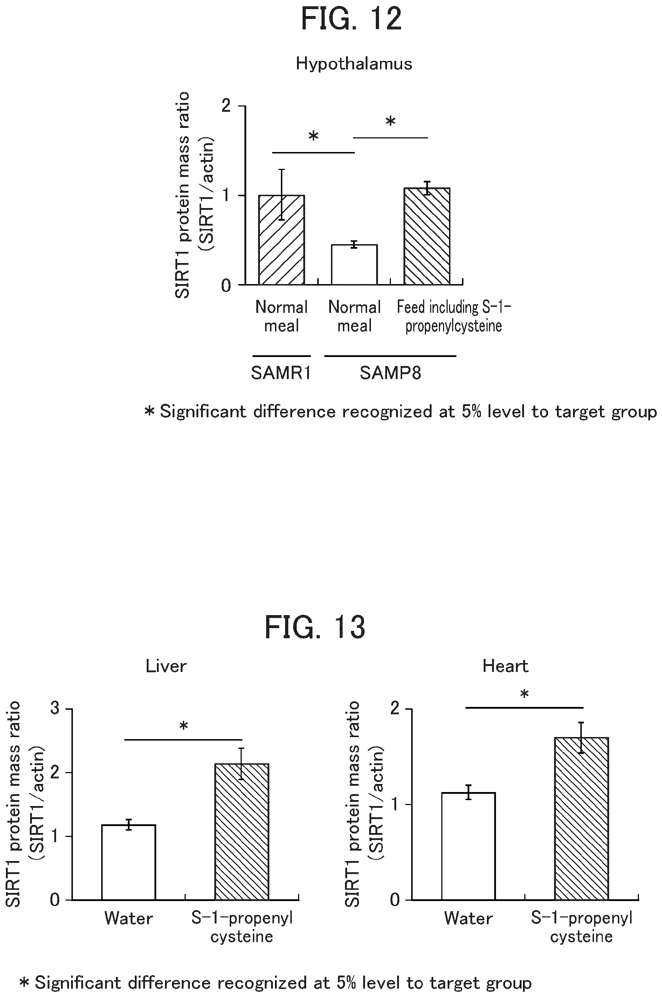

S-1-propenylcysteine (cis/trans mixture) produced in Preparation Example 2 was dissolved at a concentration of 0.001% in purified water to prepare an administration liquid. Animals for evaluating the biological activity were bred as follows. Senescence-accelerated mice SAMR1 (male) and SAMP8 (male) for testing were purchased from Japan SLC, Inc. After purchase, the animals were acclimatized for one week and used as animals for the evaluation tests. These animals for the evaluation tests were fed with regular diet and SAMP8 were fed with the diet mixed with the sample prepared above for 10 months, and then hypothalamuses of such animals were excised. After the hypothalamuses were crushed using an automatic tissue crusher, the cells were lysed with a RIPA cell lysis buffer manufactured by Millipore Corporation which was diluted 10-fold with purified water added with combined protease/phosphatase inhibitors manufactured by Thermo Fisher Scientific K.K., and then centrifuged (10,000 rpm, 10 min, 4°C), and the supernatant was used as a cell extract. The cell extract was used for analysis by Western Blotting according to a conventional method. An anti-SIRT1 antibody (manufactured by Biolegend) and an anti-β-actin antibody (manufactured by Fujifilm Wako Pure Chemical Corporation) were used as antibodies. The results are shown in FIG. 12. S-1-propenylcysteine increased the SIRT1 protein mass.

### (13) SIRT1 Protein Amount-Increasing Effect in Liver and Heart (in vivo):

After repeated oral administration of the sample prepared in the above (1)(a) to evaluation test animals prepared in the same manner as in (12) for two weeks, livers and hearts of such animals were excised. After the livers and hearts were crushed using an automatic tissue crusher, the cells were lysed with a RIPA cell lysis buffer manufactured by Millipore Corporation which was diluted 10-fold with purified water added with combined protease/phosphatase inhibitors manufactured by Thermo Fisher Scientific K.K., and then centrifuged (10,000 rpm, 10 min, 4°C), and the supernatant was used as a cell extract. The cell extract was used for analysis by Western Blotting according to a conventional method. An anti-SIRT1 antibody (manufactured by Biolegend) and an anti-β-actin antibody (manufactured by Fujifilm Wako Pure Chemical Corporation) were used as antibodies. Results are shown in Fig. 13. S-1-propenylcysteine increased the SIRT1 protein mass.

## Claims

1. An agent for sirtuin activation or sirtuin expression enhancement, an agent for increasing NAD⁺, or an agent for suppressing senescent cells, the agent comprising a compound represented by the following general formula 1A or 1B (excluding S-1-propenylcysteine) or a salt thereof as an active ingredient: 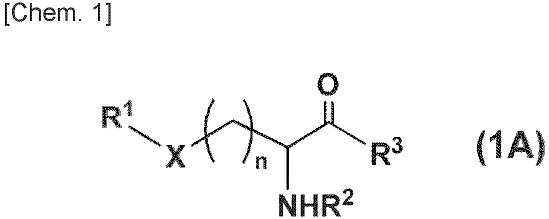
wherein R¹ represents an alkyl group that may have a substituent, an alkenyl group that may have a substituent, or an alkynyl group that may have a substituent, R² represents a hydrogen atom or an acyl group, R³ represents a hydroxy group or a monosubstituted amino group, X represents a group represented by -S-, -S(O)-, -S(O)₂-, -S-S-, -S-S-S-, or -S-S-S-S-, and n represents 1 or 2, and 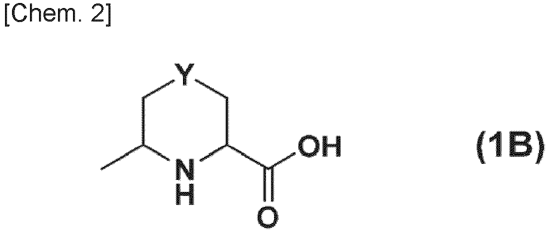
wherein Y represents a group represented by -S- or -S(O)-.

2. The agent according to claim 1, wherein in the general formula 1A,
R¹ represents a C1 to C8 alkyl group that may have a substituent, a C2 to C8 alkenyl group that may have a substituent, or a C2 to C8 alkynyl group that may have a substituent,
R² represents a hydrogen atom, a C1 to C3 alkylcarbonyl group, or a residue obtained by removing -OH from a carboxy group of an amino acid, and
R³ represents a hydroxy group or a residue obtained by removing one hydrogen atom from an amino group of an amino acid.

3. The agent according to claim 2, wherein in the general formula 1A,
R¹ represents a C1 to C8 alkyl group, a hydroxy C1 to C8 alkyl group, a C2 to C8 alkenyl group, or a C2 to C8 alkynyl group,
R² represents a hydrogen atom,
R³ represents a hydroxy group, and
X represents a group represented by -S-, -S(O)- or -S-S-.

4. The agent according to claim 1, wherein in the formula 1B,
Y represents a group represented by -S-.

5. The agent according to claim 1, wherein the compound is a compound represented by any of the following structural formulae.
